# EUROPEAN PATENT SPECIFICATION

(11) **EP 0 426 672 B1**
(45) Date of publication and mention of the grant of the patent: **05.10.1994**
(21) Application number: 89903667.7
(22) Date of filing: 22.03.1989
(51) Int. Cl.: A61M 5/315, A61M 5/32, A61M 5/34

(54) **SINGLE-USE SYRINGE**
WEGWERFSPRITZE
SERINGUE A JETER

(30) Priority: 18.04.1988 AU 7792/88; 28.10.1988 US 263705
(43) Date of publication of application: 15.05.1991
(73) Proprietor: NUJENKO PTY LTD, Myaree, W.A. 6154 (AU)
(72) Inventor: ALTER, Konrad, George, Maylands, W.A. 6051 (AU); GRIFFITHS, Jennifer, Dale, West Leederville, W.A. 6007 (AU)
(74) Representative: Pacitti, Pierpaolo A.M.E.
(86) International application number: AU8900115
(87) International publication number: WO8910151

(56) References cited:
- EP-A- 0 287 950
- AU-A- 8 536 /52
- AU-A-11 436 /88
- AU-A-13 088 /88
- AU-A-21 012 /53
- DE-A- 643 616
- FR-A- 1 005 576
- FR-A- 1 039 984
- GB-A- 789 027
- US-A- 1 648 308
- US-A- 2 457 859
- US-A- 2 474 496
- US-A- 2 502 639
- US-A- 4 643 199
- US-A- 4 692 156
- US-A- 4 747 830

## Description

THIS INVENTION relates to syringes and more particularly syringes intended for single use only.

Throughout the specification the term "syringe" shall be taken to include a hypodermic syringes and other device incorporating a needle for injecting fluid into, or extracting fluid from, a body.

Syringes are commonly intended for single use only and disposal after such use, as any subsequent use carries with it the possibility of transmission of infection by the needle of the syringe. Nevertheless there are instances where single-use syringes are used again contrary to advice from health authorities, and there is a need for a syringe which can be used only once.

A proposal for such a syringe is presented in US Patent No. 4,692,156 (Haller) in which there is disclosed a hypodermic syringe in which the barrel has a section formed of deformable plastic so that it can be bent after injection. While the primary purpose of the arrangement is to prevent complete removal of the piston and cannula assembly after the assembly has been retracted to withdraw the cannula into a protective position within the barrel, it does also serve the purpose of limiting the syringe to a single-use situation.

It is an object of this invention to provide a syringe which can be effectively rendered inoperative after use and which provides a useful choice to the earlier proposal referred to above.

Broadly, the invention resides in a syringe comprising a barrel, a plunger receivable in the barrel and movable therealong, the plunger including a shank, characterised in that said shank has a selectively deformable portion for rendering the plunger inoperable and said barrel includes a side wall having an aperture which opens onto an end of the barrel beyond which the shank extends, said aperture being adapted to receive said deformable portion of the shank upon lateral deflection of the outer end portion of the shank during deformation of the shank.

The shank may include a weakened section at the deformable portion which can be fractured to effect deformation of the shank.

The weakened section of the shank may be adapted to be fractured upon application of a predetermined bending force to the shank.

Conveniently, the weakened section is of reduced cross-sectional area in comparison to the remainder of the shank. Alternatively or additionally, the weakened section may include at least one cavity or other means for providing a zone of weakness in the weakened section.

The portion of the shank receivable in said aperture may be a portion of the weakened section of the shank.

The aperture in the side wall of the barrel may be configured to inhibit removal of the shank once it is received fully within the aperture.

In additional to preventing reuse of a syringe, it is beneficial to provide the syringe with a feature whereby the needle can be retracted into the barrel after use.

The purpose of retracting the needle into the barrel is to ensure that a person handling the syringe is protected from inadvertent wounding by the contaminated needle. This reduces the likelihood of transmission of infection by a contaminated needle.

To this end, the syringe preferably comprises a needle portion having a base and a needle mounted on the base and projecting from the base, securing means for releasably securing the base of the needle portion to the barrel with the needle extending outwardly from the barrel and the bore in the needle communicating with the interior of the barrel, said securing means being arranged to effect release of said base from the barrel upon rotation of the base relative to the barrel, an engaging means for releasably engaging the plunger with said base whereby when so engaged with the base the plunger can be rotated to effect release of the base from the barrel and then be retracted to move the needle into the region within the barrel.

Preferably, the securing means comprises screw means for threadingly engaging the base to the barrel. The screw means preferably comprises a multiple thread.

The engaging means preferably comprises a socket provided on the base of the needle portion and a projection provided on the plunger for reception in the socket and engagement therewith.

Preferably, the socket is aligned with the needle so as to provide communication between the needle and the interior of the barrel.

Engagement between the socket and the projection is effected by pushing the plunger to insert the projection into the socket.

The invention will be better understood by reference to the following description of one specific embodiment thereof as shown in the accompanying drawings in which:-
Fig. 1 is a sectional view of a syringe according to the embodiment with the needle portion shown secured to the barrel;
Fig. 2 is a view similar to Fig. 1 except that the needle portion is shown detached from the barrel and partially retracted into the barrel;
Fig. 3 is a sectional elevational view of the syringe shown during drawing of fluid into the barrel for injection into a patient at a later stage;
Fig. 4 is a sectional elevational view of the syringe shown prior to injection of the fluid contained in the barrel;
Fig. 5 is a sectional elevational view of the syringe shown after injection of the fluid has been completed and prior to retraction of the needle into the barrel;
Fig. 6 is a sectional elevational view of the syringe shown after retraction of the needle into the barrel and prior to deformation of the shank of the plunger;
Fig. 7 is a view similar to Fig. 6 with the exception that the shank of the plunger is shown partially fractured;
Fig. 8 is a sectional elevational view of the syringe shown after fracturing of the shank (with the outer section of the shank shown removed for the purpose of clarity of illustration);
Fig. 9 is a sectional elevational view of the barrel of the syringe;
Fig. 10 is an elevational view of the plunger of the syringe; and
Fig. 11 is a partly sectioned elevational view of the needle portion of the syringe.

The embodiment shown in the drawings is directed to a hypodermic syringe intended for single use only and then disposal. The syringe comprises a syringe barrel 11, a plunger 13, a needle portion 15 and a filling needle portion 17.

The barrel 11 has a nozzle 19 at the distal end 21 thereof and is open at the proximal end 23. A flange 25 is formed around the opening at the proximal end 23 of the barrel to provide a means by which a user may grip the barrel while using the syringe. The nozzle 19 defines an axial passage 27 one end of which communicated with the interior of the barrel while the other end opens to the exterior of the barrel. An internal thread formation 29 comprising multiple threads is provided on the wall of the passage 27, the purpose of which will become apparent later.

The needle portion 15 is detachably mountable on the barrel. The needle portion includes a base 31 and a hollow needle 33 mounted onto the base. The needle 33 has a sharp-pointed end 35 opposite the base 11. The base 31 is adapted to be received within the axial passage 27 defined within the nozzle of the barrel and has protrusions 28 for threadingly engaging the internal thread formation 29 provided on the wall of the passage 27.

The base 31 is hollow and when engaged in position within the nozzle of the barrel provides for fluid communication between the hollow needle 33 and the interior of the barrel.

When the needle portion 15 is to be mounted onto the barrel 11, it is inserted into the barrel through the opening at the proximal end 23 and advanced along the barrel and into the axial passage 27 of the nozzle where the base can be threadedly engaged with the nozzle.

The base 31 is provided with a hub 37 which is adapted to receive the filling needle portion. The filling needle portion is intended to locate over the hollow needle 33 and is used to draw fluid out of a vial or other container and into the syringe. The filling needle portion includes a base 39 which is adapted for location on the hub 37 and a hollow needle 41 which can be received around the hollow needle 33. The hollow needle 41 has a sharp-pointed end 43 which extends beyond the sharp-pointed end 35 of the needle 33, as shown in the drawings. The filling needle portion 17 is fitted into position on the needle portion 15 when injection fluid is to be drawn into the syringe and is removed after the filling operation, the needle portion 15 being used in the injection stage. The purpose of the filling needle portion 17 is to maintain the sterile state of the needle portion 15 during the filling operation.

The plunger 13 comprises a piston 51 and a shank 53. The piston 51 is received in the syringe barrel 11 and is in sliding and sealing engagement with the barrel. The shank 53 is connected to the piston 51 and extends out through the opening at the proximal end of the syringe barrel. The outer end of the shank 53 has a flange 55 to facilitate manual operation of the plunger.

The shank 53 is of a generally cruciform shape in cross-section except for a weakened section 57 at a region along the length of the shank. In the illustrated embodiment, the weakened section is located towards the piston end of the shank. The weakened section 57 is of reduced width in relation to the remainder of the shank and is provided with recesses 56 in its surface to enhance its weakened condition. The purpose of the weakened section 57 is to allow the shank to be fractured upon the application of a bending force to it. Fracturing of the shank effectively renders the plunger inoperable.

A cavity 58 is provided in the shank and opens onto the free end thereof for storage of the filling needle portion 17 after use.

An aperture 59 is provided in the side wall of the syringe barrel 11 and opens onto the proximal end 23 of the syringe barrel. The purpose of the aperture is to receive portion of the weakened section 57 of the shank 53 upon lateral deflection of the shank as it is being fractured. The aperture is of a configuration which inhibits removal of the shank from the aperture once received fully therein; in this embodiment such configuration is of generally keyhole shape.

The face of the piston opposite the shank is provided with an axial projection 61 adapted for reception in a socket 63 defined within the hollow base 31 of the needle portion 15. The axial projection 61 is of generally conical form and the socket 63 is of complementary shape to snugly receive the projection. The tapered surfaces of the projection 61 and the socket 63 are provided with splines 65 and 67 respectively which mesh when the projection 61 is received within the socket 63. With the projection 61 received in the socket 63, rotational torque produced by rotating the plunger about its longitudinal axis is transmitted through the projection and socket to the base 31 of the needle portion. The rotational torque transmitted to the base 31 of the needle portion effects rotation of the base to unscrew the needle portion from the nozzle. Once the needle portion has been unscrewed from the nozzle, withdrawal of the plunger retracts the needle portion into the barrel.

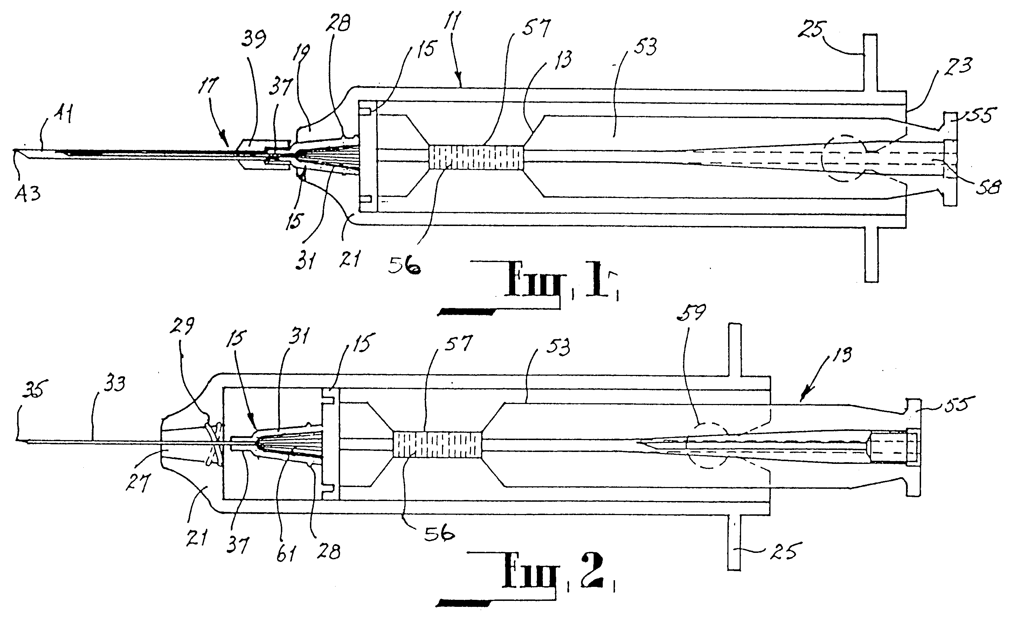

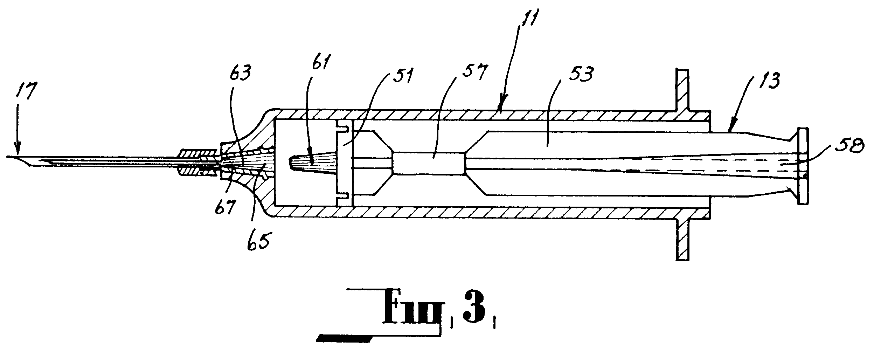

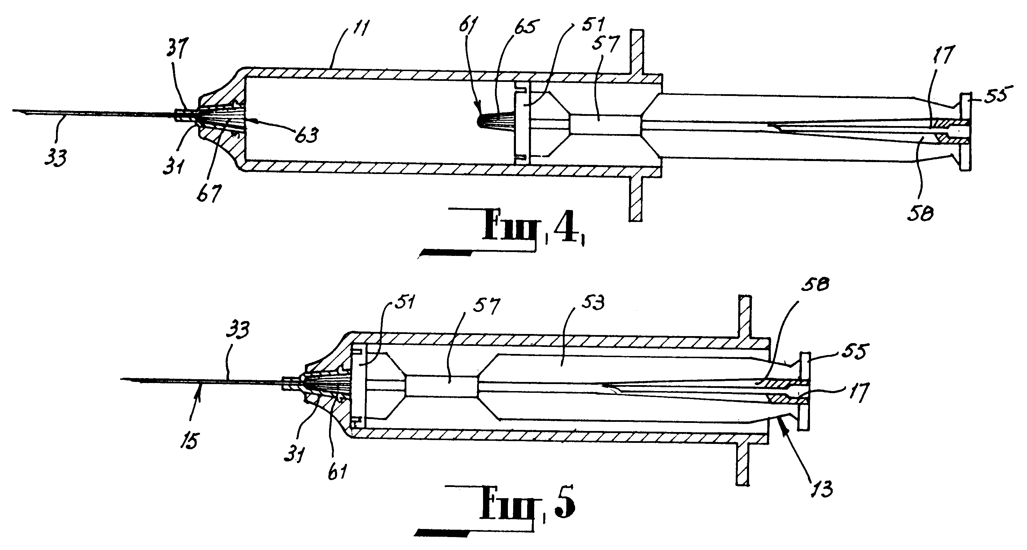

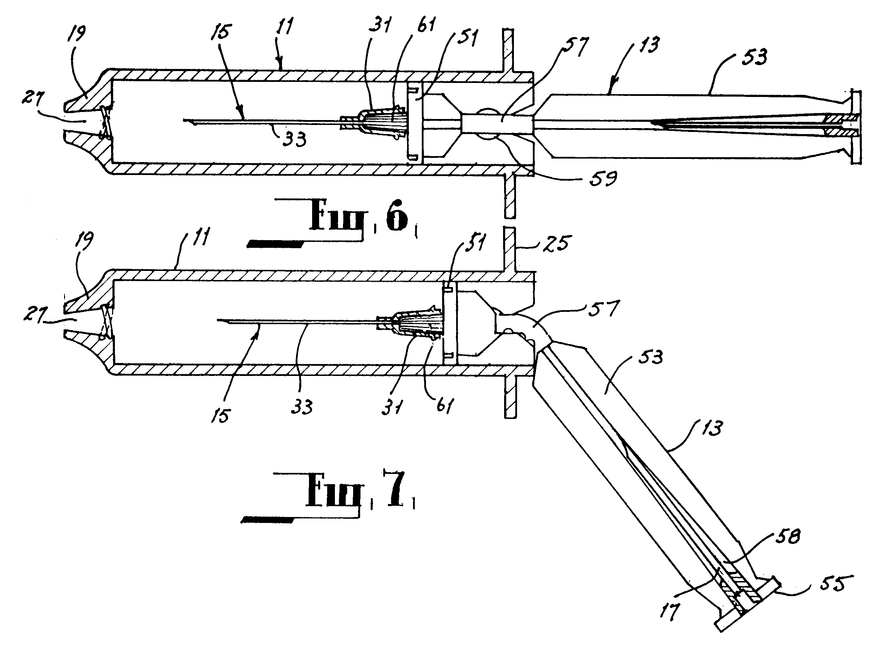

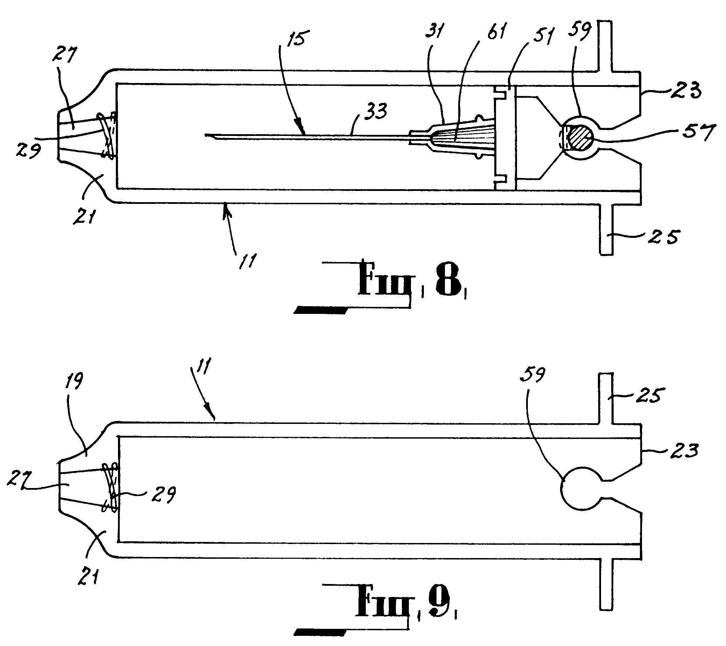

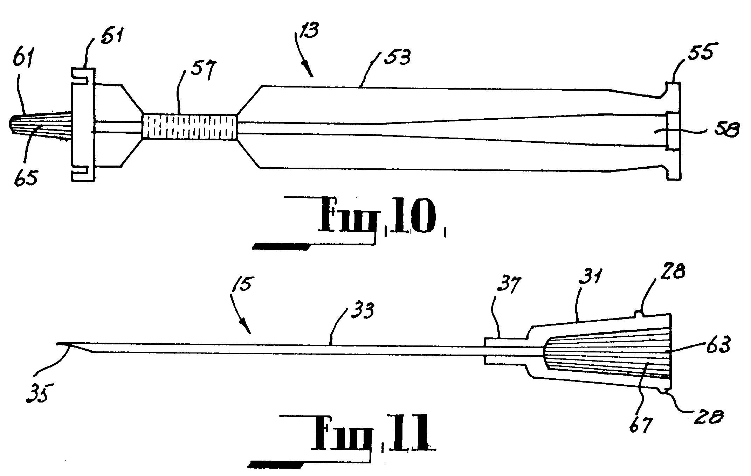

Operation of the syringe will now be described in relation to Figs. 3 to 8 of the accompanying drawings. Fluid for injection into the body of a patient is drawn into the syringe with the filling needle portion 17 in position, by retracting the plunger 13, as shown in Fig. 3. The filling needle portion is then removed from around the needle portion 15 and stored in the cavity 58 within the free end of the shank. The syringe is at this stage in readiness for injection of the fluid previously drawn into the barrel, as shown in Fig. 4. The needle 33 is inserted into the body of the patient and the plunger pushed inwardly thereby to force the injection fluid through the hollow base 31 and needle 33 into the body of the patient. As the piston 51 approaches the distal end of the syringe barrel 11 towards the end of the injection stage, the projection 61 enters the socket 63. By the end of the injection stage, the projection is snugly received in the socket, as shown in Fig. 5. The plunger is then rotated in the direction appropriate to unscrew the protrusions 28 on the base 31 from the thread formation 29 in the nozzle. After the base has been unscrewed from the nozzle, the plunger (which remain connected to the base of the needle portion ) is retracted to withdraw the needle portion 15 into the syringe barrel, as shown in Fig. 6. The plunger is then positioned such that the weakened section 57 of the shank 53 is located adjacent the aperture 59 in the syringe barrel and a bending force is applied to the shank in the direction towards the aperture, as shown in Fig. 7. The resulting lateral deflection of the shank causes the weakened section to fracture and enter the aperture, as shown in Fig. 8. The keyhole shape of the aperture serves to inhibit removal of the shank from the aperture and so the plunger is effectively locked to the syringe barrel, with the needle 33 being confined within the barrel where it is not exposed for inadvertent puncturing of the patient or any person handling the syringe.

Because the shank of the plunger has been fractured, the syringe cannot be readily used again and thus the possibility of transmission of infection by the contaminated needle is avoided.

It should be appreciated that the scope of the invention is not limited to the scope of the embodiment described, but to the scope defined by the following claims.

## Claims

1. A syringe comprising a barrel (11), a plunger (13) receivable in the barrel and movable therealong, the plunger (13) including a shank (53), characterised in that said shank (53) has a selectively deformable portion (57) for rendering the plunger (13) inoperable and said barrel (11) includes a side wall having an aperture (59) which opens onto an end (23) of the barrel (11) beyond which the shank (53) extends, said aperture (59) being adapted to receive said deformable portion (57) of the shank (53) upon lateral deflection of the outer end portion of the shank during deformation of the shank.

2. A syringe according to claim 1 wherein the shank (53) includes a weakened section (57) at the deformable portion which can be fractured upon deformation of the shank.

3. A syringe according to claim 2 wherein the weakened section (57) of the shank (53) is adapted to be fractured upon application of a predetermined bending force to the shank.

4. A syringe according to claim 2 or 3 wherein the weakened section (57) is of reduced cross-sectional area in comparison to the remainder of the shank.

5. A syringe according to claim 2, 3 or 4 wherein the weakened section (57) includes at least one cavity (56) or other means for providing a zone of weakness in the weakened section.

6. A syringe according to any one of claims 2 to 5 wherein said portion of the shank (53) receivable in said aperture (59) is a portion of the weakened section (57) of the shank.

7. A syringe according to any one of the preceding claims wherein the aperture (59) in the side wall of the barrel (11) is configured to inhibit removal of the shank (53) once it is received fully within the aperture (59).

8. A syringe according to claim 7 wherein the aperture (59) is of generally keyhole shape.

9. A syringe according to any one of the preceding claims further comprising a needle portion (15) having a base (31) and a needle (33) mounted on the base (31) and projecting from the base, securing means for releasably securing the base (31) of the needle portion to the barrel with the needle (33) expending outwardly from the barrel and the bore in the needle communicating with the interior of the barrel, said securing means (28,29) being arranged to effect release of said base (31) from the barrel (11) upon rotation of the base relative to the barrel, and engaging means (61,63) for releasably engaging the plunger (13) with said base (31) whereby when so engaged with the base (31) the plunger (13) can be rotated to effect release of the base (31) from the barrel (11) and then be retracted to move the needle (33) into the region within the barrel (11).

10. A syringe according to claim 9 wherein the securing means (28,29) comprises screw means for threadingly engaging the base (31) to the barrel (11).

11. A syringe according to claim 9 or 10 wherein the engaging means comprises a socket (63) provided on the base (31) of the needle portion (15) and a projection (61) provided on the plunger (13) for reception in the socket (63) and engagement therewith.

12. A syringe according to claim 11 wherein the socket (63) is aligned with the needle (33) so as to provide communication between the needle (33) and the interior of the barrel (11).

## Patentansprüche

1. Spritze mit einer Trommel (11) und einem Plunger (13) zur Aufnahme in der Trommel und zur Bewegung längs derselben, wobei der Plunger (13) einen Schaft (53) aufweist, dadurch gekennzeichnet, daß der Schaft (53) einen selektiv verformbaren Abschnitt (57) hat, um den Plunger (13) inoperabel zu machen, und die Trommel (11) eine Seitenwand mit einer Öffnung (59) aufweist, die sich gegen ein Ende (23) der Trommel (11) öffnet, über welches hinaus der Schaft (53) verläuft, wobei die Öffnung (53) zur Aufnahme des verformbaren Abschnitts (57) des Schafts (53) bei einer seitlichen Durchbiegung des äußeren Endabschnittes des Schafts während der Verformung des Schafts angepaßt ist.

2. Spritze nach Anspruch 1, bei welcher der Schaft (53) einen geschwächten Querschnitt (57) an dem verformbaren Abschnitt aufweist, welcher bei der Verformung des Schafts gebrochen werden kann.

3. Spritze nach Anspruch 2, bei welcher der geschwächte Querschnitt (57) des Schafts (53) dafür angepaßt ist, bei Anwendung einer vorbestimmten Biegekraft auf den Schaft gebrochen zu werden.

4. Spritze nach Anspruch 2 oder 3, bei welcher der geschwächte Querschnitt (57) eine verringerte Querschnittsfläche im Vergleich zu dem Rest des Schafts aufweist.

5. Spritze nach Anspruch 2, 3 oder 4, bei welcher der geschwächte Querschnitt (57) wenigstens einen Hohlraum (56) oder eine andere Einrichtung aufweist, um eine Schwächungszone in dem geschwächten Querschnitt zu schaffen.

6. Spritze nach einem der Ansprüche 2 bis 5, bei welcher der in der Öffnung (59) aufnehmbare Abschnitt des Schafts (53) ein Abschnitt des geschwächten Querschnitts (57) des Schafts ist.

7. Spritze nach einem der vorhergehenden Ansprüche, bei welcher die Öffnung (59) in der Seitenwand der Trommel (11) für die Behinderung einer Entfernung des Schafts (53) ausgebildet ist, wenn er erst einmal voll in der Öffnung (59) aufgenommen ist.

8. Spritze nach Anspruch 7, bei welcher die Öffnung (59) die generelle Form eines Schlüssellochs hat.

9. Spritze nach einem der vorhergehenden Ansprüche, welche weiterhin einen Nadelabschnitt (15) mit einer Basis (31) und einer Nadel (33) aufweist, die an der Basis (31) befestigt ist und von der Basis vorsteht, eine Befestigungseinrichtung für eine lösbare Befestigung der Basis (31) des Nadelabschnitts an der Trommel, wobei die Nadel (33) von der Trommel nach außen verläuft und die Bohrung in der Nadel mit dem Innern der Trommel verbunden ist, wobei die Befestigungseinrichtung (28, 29) für die Ermöglichung eines Lösens der Basis (31) von der Trommel (11) durch eine Drehung der Basis relativ zu der Trommel angeordnet ist, und eine Eingriffseinrichtung (61, 63) für einen lösbaren Eingriff des Plungers (13) mit der Basis (31), wodurch der Plunger (13) bei einem solchen Eingriff mit der Basis (31) gedreht werden kann, um das Lösen der Basis (31) von der Trommel (11) zu bewirken und dann zurückgezogen zu werden, um die Nadel (33) in den Bereich innerhalb der Trommel (11) zu bewegen.

10. Spritze nach Anspruch 9, bei welcher die Befestigungseinrichtung (28, 29) eine Schraubeinrichtung für einen verschraubbaren Eingriff der Basis (31) mit der Trommel (11) aufweist.

11. Spritze nach Anspruch 9 oder 10, bei welcher die Eingriffseinrichtung einen an der Basis (31) des Nadelabschnitts (15) vorgesehenen Sockel (63) und einen Vorsprung (61) aufweist, der an dem Plunger (13) für eine Aufnahme in dem Sockel (63) und einen Eingriff damit vorgesehen ist.

12. Spritze nach Anspruch 11, bei welchem der Sockel (63) mit der Nadel (33) fluchtet, um eine Verbindung zwischen der Nadel (33) und dem Innern der Trommel (11) zu schaffen.

## Revendications

1. Seringue comprenant un cylindre (11), un piston (13) adaptable dans le cylindre et déplaçable le long de celui-ci, le piston (13) comprenant une tige (53), caractérisée en ce que ladite tige (53) comprend une portion (57) sélectivement déformable pour rendre le piston (13) inapte à fonctionner et ledit cylindre (11) comprend une paroi latérale ayant une ouverture (59) qui s'ouvre à une extrémité (23) du cylindre (11) au-delà de laquelle la tige (53) s'étend, ladite ouverture (59) étant adaptée pour recevoir ladite portion déformable (57) de la tige (53) sous l'action d'une flexion latérale de la portion d'extrémité externe de la tige pendant la déformation de la tige.

2. Seringue selon la revendication 1 dans laquelle la tige (53) comprend un tronçon affaibli (57) dans la portion déformable, qui peut être fracturé par déformation de la tige.

3. Seringue selon la revendication 2 dans laquelle le tronçon affaibli (57) de la tige (53) est adapté pour être fracturé par application sur la tige d'une force de flexion prédéterminée.

4. Seringue selon les revendications 2 ou 3 dans laquelle le tronçon affaibli (57) présente une section transversale de surface réduite par rapport au reste de la tige.

5. Seringue selon les revendications 2, 3 ou 4 dans laquelle le tronçon affaibli (57) comprend au moins une cavité (56) ou d'autres moyens pour réaliser une zone d'affaiblissement dans le tronçon affaibli.

6. Seringue selon l'une quelconque des revendications 2 à 5 dans laquelle ladite portion de tige (53) adaptable dans ladite ouverture (59) est une portion du tronçon affaibli (57) de la tige.

7. Seringue selon l'une quelconque des revendications précédentes dans laquelle l'ouverture (59) dans la paroi latérale du cylindre (11) est configurée pour empêcher l'enlèvement de la tige (53) une fois qu'elle est introduite entièrement dans l'ouverture (59).

8. Seringue selon la revendication 7 dans laquelle l'ouverture (59) présente la forme générale d'un trou de serrure.

9. Seringue selon l'une quelconque des revendications précédentes comprenant en outre une portion d'aiguille (15) ayant une base (31) et une aiguille (33) montée sur la base (31) et se développant depuis la base, un moyen de fixation pour fixer de manière détachable la base (31) de la portion d'aiguille au cylindre avec l'aiguille (33) s'étendant vers l'extérieur depuis le cylindre et le trou dans l'aiguille communiquant avec l'intérieur du cylindre, ledit moyen de fixation (28, 29) étant adapté pour assurer le dégagement de ladite base (31) par rapport au cylindre (11) par rotation de la base par rapport au cylindre, et un moyen d'engagement (61, 63) pour engager de façon détachable le piston (13) avec ladite base (31) de sorte que lorsqu'il est ainsi engagé avec la base (31) le piston (13) peut être tourné pour assurer le dégagement de la base (31) par rapport au cylindre (11) et peut alors être retiré pour déplacer l'aiguille (33) et l'introduire à l'intérieur du cylindre (11).

10. Seringue selon la revendication 9 dans laquelle le moyen de fixation (28, 29) comprend un moyen de vis pour engager par vissage la base (31) sur le cylindre (11).

11. Seringue selon les revendications 9 ou 10 dans laquelle le moyen d'engagement comprend un embout femelle (63) prévu sur la base (31) de la portion d'aiguille (15) et une projection (61) prévue sur le piston (13) pour être reçue dans l'embout femelle (63) et s'engager avec lui.

12. Seringue selon la revendication 11 dans laquelle l'embout femelle (63) est aligné avec l'aiguille (33) pour assurer la communication entre l'aiguille (33) et l'intérieur du cylindre (11).
